# EUROPEAN PATENT APPLICATION

(11) **EP 4 201 411 A1**
(43) Date of publication of application: **28.06.2023**
(21) Application number: 21216700.1
(22) Date of filing: 21.12.2021
(51) Int. Cl.: A61K 31/714, A61P 17/16, A61P 37/00, A61Q 17/04

(54) **NOVEL USE OF VITAMIN B12**

(71) Applicant: DSM IP Assets B.V., 6411 TE Heerlen (NL)
(72) Inventor: SCHUETZ, Rolf, 4303 Kaiseraugst (CH)
(74) Representative: Berg, Katja

(57) **Abstract**

The present invention relates to the use of Vitamin B12 as NLRP3 inflammasome inhibitor as well as to the prevention or treatment of ailments in which NLRP3 signaling contributes to the pathology, and/or symptoms, and/or progression, of said ailments.

## Description

The present invention relates to the use of Vitamin B12 as NLRP3 inflammasome inhibitor as well as to the prevention or treatment of ailments in which NLRP3 signaling contributes to the pathology, and/or symptoms, and/or progression, of said ailments.

NOD-like receptor proteins (NLRP) and the inflammasome are currently very prominent topics in biology, including skin health. Recent studies have shown that UV-radiation and environmental air pollutants can induce NLRP3-inflammasome activity and thus contribute to skin damage such as skin ageing. Thus, more and more cosmetic ingredients targeting this mechanism are being introduced into the marketplace.

It has been well established by now, that both microRNAs miR-22-3p and miR-30e-5p play a significant role in the regulation of the inflammasome. Both microRNAs are able to inhibit messenger RNA (mRNA) and protein expression of NLRP3 and thus reduce inflammasome activity by inhibiting the NLRP-mediated activation of caspase 1 (CASP1) and mature IL-1β.

Thus, there is an ongoing need for cosmetically acceptable ingredients which act as NLRP3-inflammasome inhibitor and can thus be used to counteract UV-radiation and/ or environmental air pollution-induced skin damage to ameliorate the health and appearance of the skin.

Surprisingly it has now been found that vitamin B12 produced a statistically significant increase in expression of the two microRNAs with fold change values greater than 2.0 and is thus able to reduce inflammasome activity in skin tissue exposed to UV-radiation and/ or environmental air pollutants and accordingly to mitigate skin damages resulting thereof. This effect has been further illustrated by topically applied vitamin B12 on UV-irradiated skin explants. The vitamin B12 treatment showed a significant upregulation of miR-22-3p expression, a significant decrease in the protein component ASC (apoptosis-associated speck-like protein containing a caspase activation and recruitment domain) of the inflammasome complex, and a significant reduction in IL1-β after UV-induced stress on skin explants compared to an untreated control. ASC is a protein containing a PYCARD-specific motif that is involved in the assembly of inflammatory complexes and apoptotic signaling via caspase activation and therefore a key marker of the inflammasome. The secretion of Interleukin-1β is the response to activation of the stress-induced inflammatory signaling pathway.

Thus, in a first embodiment, the present invention relates to a composition comprising an effective amount of vitamin B12 for its use in a dermatological treatment as agent for protecting skin cells against NLRP3-inflammasome activation, in particular NLRP3-inflammasome activation caused by ultraviolet radiation and/ or air pollutants.

In another embodiment, the present invention concerns a cosmetic or dermatological composition comprising an effective amount of vitamin B12 for use in the prevention or treatment of skin conditions caused by environmental factors, in particular UV-radiation (UV-R) and/ or air pollution-induced increase in inflammasomes and optionally appreciating the effect.

In a further embodiment, the present invention relates to a method to prevent or treat skin conditions caused by UV-radiation and/ or air pollution-induced increase in inflammasomes comprising the following steps:
a) providing a cosmetic or dermatological composition comprising vitamin B12, and
b) applying an amount of said composition to human skin or scalp that is sufficient to mitigate the inflammasome activation and control the inflammasome homeostasis.

The inhibitory effect of vitamin B12 on NLRP3 inflammasome according to the present invention is understood to reduce or inhibit activation of caspase 1 (CASP1) and in turn cleavage of pro-IL1B and pro-IL18 into active forms, by mitigating an inflammatory cascade.

In a further aspect, the present invention relates to vitamin B12 (and/or cosmetic or dermatological compositions comprising vitamin B12) for: the use in the prevention and/ or treatment of a skin condition associated with NLRP3 activity (including inflammasome activity); in the treatment of a skin condition in which the NLRP3 signaling contributes to the pathology, and/or symptoms, and/or progression, of said skin condition, in inhibiting NLRP3 inflammasome activity (including in a subject in need thereof), and/or as an NLRP3 inhibitor. Specific skin conditions are e.g., irritated skin, sensitive skin, skin erythema, and skin ageing.

In another aspect, there is provided a use of vitamin B12 (and/or cosmetic or dermatological compositions comprising vitamin B12): in the treatment of a disease or disorder associated with NLRP3 inflammasome activity; in the treatment of a disease or disorder in which the NLRP3 inflammasome signaling contributes to the pathology, and/or symptoms, and/or progression, of said disease/disorder; in inhibiting NLRP3 inflammasome activity (including in a subject in need thereof), and/or as an NLRP3 inhibitor.

In another aspect, there is provided a use of vitamin B12 (and/or cosmetic or dermatological compositions comprising Vitamin B12) in the manufacture of a medicament for: the treatment of a disease or disorder associated with NLRP3 inflammasome activity; the treatment of a disease or disorder in which the NLRP3 inflammasome signaling contributes to the pathology, and/or symptoms, and/or progression, of said disease/disorder, and/or inhibiting NLRP3 inflammasome activity (including in a subject in need thereof).

In another aspect, there is provided a method of treating a disease or disorder in which the NLRP3 inflammasome signaling contributes to the pathology, and/or symptoms, and/or progression, of said disease/disorder, comprising administering an effective amount of vitamin B12, for instance to a subject (in need thereof).

The present invention also relates to vitamin B12 as NLRP3 inflammasome inhibitor.

The term 'skin' as used in this document, is meant to include the external surface of mammals, especially humans and includes the skin and the scalp. Preferred skin in all embodiments of the present invention is facial and body skin such as most preferably facial skin.

The term 'prevention' as used herein refers to lessening the risk of developing a skin condition associated with NLRP3 inflammasome activity such as skin-ageing.

The term 'treatment' as used herein refers to an amelioration of symptoms, delaying the onset and/ or reduction of the duration of any diseases associated with NLRP3 inflammasome activity. The treatment can be prophylactic (cosmetic) or therapeutic. Preferably, the treatment is prophylactic.

The term 'NLRP3' refers to the 'NOD-like receptor family pyrin domain containing 3'. NLRP3 is a component of the innate immune system that functions as cytoplasmic sensor that recognizes pathogen- and damage-associated molecular patterns. When NLRP3 recognizes its ligand, it recruits an adaptor, apoptosis associated speck containing a caspase activation and recruitment domain (ASC). NLRP3 together with the adaptor ASC protein forms a caspase-1 activating complex known as the NLRP3 inflammasome. The NLRP3 inflammasome cleaves and thus activates caspase-1 which in turn can initiate the maturation of interleukin 1 β (I L-1 β) and IL-18 by cleaving their inactive precursors. Activation of these interleukins allows to activate the inflammatory pathway of cell death, called pyroptosis.

The term 'NLRP3 inflammasome inhibitor' refers to a compound capable of inhibiting the activation of the inflammasome via the NLRP3 receptor belonging to the NOD-like receptor (NLR) family. More particularly, vitamin B12 is capable of inhibiting the activation of the inflammasome via the NLRP3 by inhibiting the activation of IL1-β by the caspase 1 pathway.

The term 'effective amount' as used herein refers to an amount necessary to obtain the physiological effect. The physiological effect may be achieved by one application dose or by repeated applications. The dosage administered may, of course, vary depending upon known factors, such as the physiological characteristics of the particular cosmetic or dermatological composition comprising vitamin B12 and its mode and route of administration; the age, the nature and extent of the symptoms; the kind of concurrent treatment; the frequency of treatment; and the effect desired and can be adjusted by a person skilled in the art.

The term 'dermatological' as used herein may refer to cosmetic (non-therapeutic) as well as pharmaceutical (therapeutic) treatments. In all embodiments of the present invention cosmetic treatments i.e. treatments intended for beautifying the skin are preferred.

The term 'cosmetic or dermatological composition' as used herein refers to compositions, which are used to treat, care for or improve the appearance of the skin and/or the scalp. Particular advantageous cosmetic or dermatological compositions are skin care preparations.

Environmental air pollutants according to the present invention are in particular polycyclic aromatic hydrocarbons (PAHs), volatile organic compounds (VOCs), oxides, particulate matter (PM), ozone (O3), and cigarette smoke.

Vitamin B12 (INCI cyanocobalamin) is e.g. commercially available at DSM nutritional Products Ltd. as Quali^{®}-B.

In all embodiments of the present invention preferably the cells are skin cells such as in particular epithelial cells, especially keratinocytes, melanocytes, fibroblasts or dendritic cells.

In all embodiments of the present invention preferably the treatment is non-therapeutic, i.e. cosmetic.

In all embodiments of the present invention, preferably, the amount of vitamin B12 in the compositions according to the present invention is selected in the range of 0.0001 wt.-% to 0.1 wt.-%, preferably in the range of 0.001 wt.-% to 0.05 wt.-%, most preferably in the range of 0.001 to 0.025 wt.-%, based on the total weight of the composition. Further suitable ranges include 0.001 wt.% to 0.01 wt.-%.

Preferably, the amount of the cosmetic or dermatological composition according to the present invention to be applied to the skin is selected in the range of 0.1 to 3 mg/ cm² skin, such as preferably in the range of 0.1 to 2 mg/ cm² skin and most preferably in the range of 0.5 to 2 mg / cm² skin.

Preferably, in all embodiments of the present invention, vitamin B12 is suitable for use in inhibiting the effects of UV-R, preferably UV-B, exposure, or alleviating the effects of UV-R, preferably UV-B, induced damage on the skin caused by the activation of NLRP3 inflammasome.

Thus, preferably, in all embodiments of the present invention, the compositions according to the present invention, next to vitamin B12, further comprises at least one UV-R, preferably UV-A filter substance, most preferably extending protection in the visible light range.

Suitable UV filter substances absorbing UV-radiation such as in particular UV-A and/ or UV-B radiation are well known to a person skilled in the art and encompass oil soluble, water soluble and inorganic UV-filter substances.

Advantageous oil soluble UV-filter substances according to the present invention encompass dibenzoylmethane derivatives such as butyl methoxydibenzoylmethane (e.g. commercially available as PARSOL^{®} 1789 at DSM Nutritional Products Ltd), organopolysiloxanes functionalized with at least one UV-light absorbing group such as polysilicone-15 (e.g. commercially available as PARSOL^{®} SLX at DSM Nutritional Products Ltd), diphenylacrylates such as octocrylene (e.g. commercially available as PARSOL^{®} 340 at DSM Nutritional Products Ltd), cinnamates such as octyl methoxycinnamate (e.g. commercially available as PARSOL^{®} MCX at DSM Nutritional Products Ltd), triazine derivatives such as Bis-Ethylhexyloxyphenol Methoxyphenyl Triazine (commercially available as PARSOL^{®} SHIELD at DSM Nutritional Products Ltd), Ethyl Hexyl Triazone (e.g. commercially available as PARSOL^{®} EHT at DSM Nutritional Products Ltd), Diethylhexyl butamido Triazone (Uvasorb^{®} HEB), Diethylamino Hydroxybenzoyl Hexyl Benzoate (Uvinul^{™} A+), salicylates such as octylsalicylate (ethylhexyl salicylate) and homosalate (e.g. commercially available as PARSOL EHS, respectively HMS EHT at DSM Nutritional Products Ltd) as well as mixtures thereof.

The total amount of the oil soluble UV-filter substance(s) in the composition according to the invention is preferably selected in the range of about 0.1 to 25 wt.-%, more preferably in the range of about 0.5 to 20 wt.-%, most preferably in the range of 1 to 15 wt.-% based on the total weight of the composition.

Advantageous water soluble UV-filter substance according to the present invention encompass 2-phenylbenzimidazol-sulphonic acid or 2,2'-(1,4-phenylene)bis-1H-benzimidazole-5,7-disulfonic acid as well as the respective salts thereof. Preferably the water soluble UV-filter substance is 2-phenylbenzimidazol-sulphonic acid (e.g. commercially available as PARSOL^{®} HS at DSM Nutritional Products Ltd).

The total amount of the water soluble UV-filter substance(s) in the composition according to the invention is preferably selected in the range of about 0.1 to 5 wt.-%, more preferably in the range of about 0.5 to 4 wt.-%, most preferably in the range of 1 to 3 wt.-% based on the total weight of the composition

Advantageous inorganic UV-filter substances according to the present invention encompass UV-light absorbing pigments such as e.g. microparticulated Zink oxide or Titanium dioxide. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art. In all embodiment of the present invention, preferably the inorganic UV-filter substance is selected from titanium dioxide having an inner silica and an outer dimethicone coating which is commercially available as PARSOL^{®} TX at DSM Nutritional Products Ltd or from zinc oxide having a triethoxycaprilylsilane coating which is commercially available as PARSOL^{®} ZX at DSM Nutritional Products Ltd.

The total amount of the inorganic UV-filter substance(s) in the composition according to the invention is preferably selected in the range of about 0.1 to 10 wt.-%, more preferably in the range of about 0.5 to 8 wt.-%, most preferably in the range of 2 to 5 wt.-% based on the total weight of the composition.

In all embodiments of the present invention, preferably, the composition is applied on the skin before or after exposure or contact with ultraviolet radiation and/ or air pollutants.

The cosmetic or dermatological compositions according to the invention are intended for topical application, which is to be understood as the external application to keratinous substances, such as in particular the skin.

As the cosmetic or dermatological compositions according to the invention are intended for topical application, they comprise a physiologically acceptable medium, that is to say a medium compatible with keratinous substances, such as in particular the skin. In particular the physiologically acceptable medium is a cosmetically, respectably dermatologically acceptable carrier.

The term 'cosmetically acceptable carrier' respectively 'dermatologically acceptable carrier' as used herein refers to a physiologically acceptable medium which is compatible with keratinous substances. Suitable carriers are well known in the art and are selected based on the end-use application. Preferably, the carriers of the present invention are suitable for application to skin (e.g., sunscreens, creams, milks, lotions, masks, serums, hydrodispersions, foundations, creams, creamgels, or gels etc.). Such carriers are well-known to one of ordinary skill in the art and can include one or more compatible liquid or solid filler diluent, excipient, additive or vehicle which are suitable for application to skin. The exact amount of carrier will depend upon the level of the active(s), respectively active blends and any other optional ingredients that one of ordinary skill in the art would classify as distinct from the carrier (e.g., other active components). The cosmetic compositions of the present invention preferably comprise from about 70% to about 99.999%, more preferably from about 85% to about 99.99%, still more preferably from 90% to about 99%, and most preferably, from about 93% to about 98%, by weight of the cosmetic composition, of a carrier.

The cosmetic compositions of the present invention can be formulated into a wide variety of product types, including creams, waxes, pastes, lotions, milks, mousses, gels, oils, tonics, and sprays. Preferably the active(s), respectively the active blends are formulated into lotions, creams, gels, and tonics. These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If cosmetic or dermatological compositions of the present invention are formulated as an aerosol and applied to the skin as a spray-on product, a propellant is added to the cosmetic composition.

The cosmetic or dermatological compositions according to the present invention can be prepared by conventional methods in the art such as e.g. by admixing an active or an active blend according to the present with the cosmetically acceptable carrier.

The cosmetic or dermatological compositions of the invention (including the carrier) may comprise further conventional adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such cosmetic or dermatological compositions.

In accordance with the present invention, the cosmetic or dermatological compositions according to the invention may also comprise further cosmetically active ingredients conventionally used in cosmetic and/ or dermatological compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic or dermatological excipients, diluents, adjuvants, additives as well as active ingredients commonly used in the skin care industry which are suitable for use in the cosmetic or dermatological compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of further active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The further cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic or dermatological compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the cosmetic or dermatological composition is an emulsion, such as in particular an O/W, W/O, Si/W, W/Si, O/W/O, W/O/W multiple or a pickering emulsion, then the amount of the oily phase present in such cosmetic or dermatological emulsions is preferably at least 10 wt.-%, such as in the range of 10 to 60 wt.-%, preferably in the range of 15 to 50 wt.-%, most preferably in the range of 15 to 40 wt.-%, based on the total weight of the cosmetic or dermatological composition.

In one embodiment, the cosmetic or dermatological compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the cosmetic or dermatological composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the cosmetic or dermatological composition.

Particular suitable O/W emulsifiers to be used in the cosmetic or dermatological compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the cosmetic or dermatological compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to cosmetic or dermatological compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

The cosmetic or dermatological compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The following examples are provided to further illustrate the effects of the present invention. These examples are illustrative only and are not intended to limit the scope of the invention in any way.

### Examples

In a screening experiment in keratinocytes by miRNA microarray profiling and bioinformatic analysis, two miRNAs, miR-22-3p and miR-30e-5p, were identified that may target NLRP3 mRNA in inflammatory damage in skin.

Method for miRNA microarray profiling:
The study was conducted to understand how different vitamin B12 influence microRNA and mRNA gene expression in keratinocytes. MicroRNAs are small, non-coding RNA molecules that function as negative regulators of gene expression. MicroRNA expression was analyzed using hsa-miR-22 and hsa-miR-30e and RNU44 as control microRNA. The current study was performed using normal human epidermal keratinocytes [NHEK].
Vitamin B12 was diluted in DermaLife K medium. NHEK cultured in DermaLife K medium without added supplements served as the Control group. An initial cytotoxicity screening was performed to evaluate a range of vitamin B12 concentrations prior to the microRNA analysis. The test concentration for vitamin B12 was 0.03 mM.

MicroRNA expression results:
Unpaired t-tests (p<0.05, N=4) were performed to compare vitamin B12 treatment to the control group. Statistically significant changes in expression were observed. The endogenous control microRNA (RNU44) was used for normalization. In table 1, results are shown:

**Table 1: fold-change increase vs untreated control**

| microRNA | **0.03 mM vitamin B12** |
|---|---|
| hsa-miR-22 | 2.02 |
| hsa-miR-30e | 2.22 |

To further define the benefits of vitamin B12 in the skin, we included analysis of the compound in response to a stressor that induces inflammasome-medicated inflammation. Stress model options include UV exposure, confirming the effects on more complex skin tissues (ex vivo model). Firstly, the cytotoxicity of the active ingredient was evaluated. Then, the capacity of the active ingredient was measured to regulate inflammasome activation. Hence, Interleukin-1β (IL-1β, early inflammation marker) was quantified in culture medium using an ELISA kit assay. Using immunohistochemistry, the effect of the active ingredient on the level of ASC protein (inflammasome marker) was studied. Finally, the expression level of miRNA hsa-miR-22-3p was quantified from RT-qPCR.

Ex vivo culture model and treatment:
Fresh skin explants were obtained from abdominal surgery of a 52-year-old Caucasian woman. The culture medium dedicated to skin explants was used and renewed every day during each treatment. The skin biopsies were cultured in different conditions and each condition was performed in triplicates and processed separately. The active ingredient treatment was applied before and after the UV-B stress, but not during the UV stress. An UV-B irradiation of 200J/cm² for 2 minutes was applied identically onto each irradiated skin explant. Prior to the irradiation, the culture medium was removed and replace by PBS +/+ for the duration of the stress.

Samples:
1.Control Explant (No treatment, no irradiation) (NT_NS)
2. Explant + UVB (NT_UV)
3. Explant + UVB + vitamin B12 (0.01%)
4. Explant + UVB + vitamin B12 (0.001%)
Total no. of samples = 4 x 3 = 12 samples

Cytotoxicity method:
Lactate dehydrogenase assay kit (LDH) was purchased from Roche and used according to manufacturer's protocol.

### miRNA extraction and real-time quantitative PCR

The miRNAs were extracted and purified using miRCURY RNA Isolation Kit. The quality control and miRNA quantification were performed using Agilent RNA Small kit and Analysis Agilent 2100 bioanalyzer. For miR qPCR, the RNA was reverse transcribed with a miRCURY LNA RT kit (339340, Qiagen). The reverse transcription reactions were diluted, and a fraction of diluted cDNA was used for each quantitative PCR, which was performed with a miRCURY LNA SYBR Green PCR kit (339346, Qiagen) using the CFX-connect (Biorad). The results were normalized to U6 expression. The reference and expression level of miRNA were calculated using 2ΔΔCt method.

### IL-1β competitive ELISA

The secretion of Interleukin-1β in response to activation of the stress-induced inflammatory signaling pathway was evaluated using a competitive ELISA assay. 24 hours after exposure to UV-B irradiation, culture supernatants of explants were recovered. The samples were then treated according to the manufacturer's instructions using the IL-1β (RAB0273, Sigma Aldrich). The Interleukin production by explants was measured at 450 nm using ENSIGHT.

### ASC immunostaining

To evaluate the onset of apoptotic signaling, the fluorescence intensity of ASC was measured. After UV exposure, the explants were replaced in a fresh culture medium. At the end of the treatments the explants were frozen and then cut into thin sections. Following this, immunohistochemistry was performed by adding primary monoclonal Antibody ASC/TMS1/PYCARD (clone B-3) (SantaCruz #sc-514414) and secondary antibody anti-mouse coupled to Cy5 (Jackson Laboratory). After Hoechst 33342 staining, the fluorescence intensity was measured using a Zeiss fluorescence microscope (Axio imager Z1).

### Results from ex vivo experiments

### Cytotoxicity results

Table 1 shows that stress and treatment did not exert cytotoxicity compared to the biopsies treated with Triton 0.1%.

**Table 1: Effect of vitamin B12 on LDH production by skin explants at 2 concentrations (0,01% and 0,001%) and of the positive control Triton at 0.1% after irradiation with 200 mJ/cm² UVB. The statistical analysis was performed using two-tailed unpaired T-test and was not significant (p<0.05) for the vitamin B12 treatment vs irradiated control (NT_UV).**

| **Samples name** | **Cytotoxicity [OD 450 nm] Mean/conditions** | **SEM** |
|---|---|---|
| NT NS 1 | 0.184 | 0.056 |
| NT NS 2 | | |
| NT NS 3 | | |
| NT UV 2 | 0.145 | 0.053 |
| NT UV 3 | | |
| NT UV 4 | | |
| Vit B12_0,01%_UV 1 | 0.131 | 0.030 |
| Vit B12_0,01%_UV 2 | | |
| Vit B12_0,01%_UV 4 | | |
| Vit B12_0,001%_UV 1 | 0.163 | 0.096 |
| Vit B12_0,001%_UV 3 | | |
| Vit B12_0,001%_UV 4 | | |
| Tx100 | 1.549 | 0.245 |
| Tx100 | | |

### MicroRNA miR-22-3p quantification results

Table 2 shows that the expression level of miR-22-3p is significantly increased by the vitamin B12 treatment at 0.01%. At 0,001% the active ingredient tends to increase miR-22-3p expression, but this activation is not statistically significant.

**Table 2: Effect of vitamin B12 on miR expression in skin explants after UV-B stress. The explants were treated with vitamin B12 at two concentrations (0,01% and 0,001%) and irradiated with UV-B at 200 mJ/cm². The miR expression was normalized using U6 RNA. Two-tailed unpaired t-test was used for statistical analysis (using values from 3 replicates) vs NT_UV (*p-value <0.05).**

| **miR-22-3p** | QR mean | SEM | **STAT_T-Test 2-2 vs NT_UV (basal effect)** |
|---|---|---|---|
| NT_NS 2 | 1.015 | 0.126 | 0.742 |
| NT_NS 3 | | | |
| NT_NS 4 | | | |
| NT_UV 2 | 1.067 | 0.004 | 1 |
| NT_UV 3 | | | |
| NT_UV 4 | | | |
| Vit B12_0,01%_UV 1 | 1.721 | 0.287 | 0.040^{*} |
| Vit B12_0,01%_UV 2 | | | |
| Vit B12 0,01% UV 3 | | | |
| Vit B12_0,001% UV 1 | 1.334 | 0.248 | 0.335 |
| Vit B12_0,001%_UV 2 | | | |
| Vit B12_0,001%_UV 3 | | | |
| Vit B12_0,001%_UV 4 | | | |

IL-1β secretion results:
UV-B increased the production of IL-1β compared to the untreated control. Vitamin B12 at 0,01% seems to decrease the IL-1β secretion after UV-B stress (Table 3).

**Table 3: Effect of vitamin B12 treatment on IL-1β secretion from skin explant after UV-B stress Statistical analysis was performed using a two-tailed unpaired t-test vs NT_UV (* p value <0.05).**

| **Sample condition** | **Mean Intensity** | **SEM** | **Relative Intensity** | **t-test vs NT_UV** |
|---|---|---|---|---|
| NT_NS 2 | 3.122 | 0.139 | 100 | 0.046* |
| NT_NS 3 | | | | |
| NT_NS 4 | | | | |
| NT_UV 1 | 4.914 | 0.615 | 157 | 1 |
| NT_UV 2 | | | | |
| NT_UV 4 | | | | |
| Vit B12_0,01%_UV 2 | 4.2644 | 0.616 | 137 | 0.497 |
| Vit B12_0,01%_UV 3 | | | | |
| Vit B12_0,01%_UV 4 | | | | |

ASC complex immunolabelling results:
Table 4 shows that vitamin B12 at 0.01% can significantly reduce the ASC complex activation to the level of the none irradiated extract, compared to the UV-B stressed sample. The active ingredient at 0.001 % also significantly reduces the ASC complex activation after UV-B treatment, but to a lower level compared to the sample containing 0.01% of active ingredient.

**Table 4: Quantification of ASC complex immunolabelled in skin explant sections with or without UV-B stress and after vitamin B12 treatment at 0,01% or 0,001% and irradiated or not with UVB at 200mJ/cm². The statistical analysis was performed using two-tailed unpaired T-test vs NT_UV (+ p-value <0.05, ++ p-value<0.01, +++ p-value<0.001).**

| **Condition (n =38)** | **mean_int (int/area)** | **Mean intensity / condition** | **%** | **SD-ERR** | **wilcoxon vs NTUV** |
|---|---|---|---|---|---|
| NTNS | 674.01 | 477.70 | 100.00 | 20.71 | 1,079E-07⁺⁺⁺ |
| NTUV | 634.81 | 660.03 | 138.17 | 15.48 | 1 |
| Vit B12_0,001%_UV | 524.44 | 565.99 | 118.48 | 19.47 | 0.0001329⁺⁺⁺ |
| Vit B12_0,01%_UV | 491.31 | 493.07 | 103.22 | 14.56 | 2.898E-09⁺⁺⁺ |

Surprisingly we found a photo-preventive effect by the application of vitamin B12 when we applied 0.001 % and 0.01 % vitamin B12 topically on UV-irradiated skin explants. IL-1β was significantly induced by UV and was reduced with vitamin B12 (0.01 %) treatment. ASC protein was induced by UV whereas vitamin B12 significantly reduced the ASC protein in a dose-dependent manner.

## Claims

1. A composition comprising an effective amount of vitamin B12 for its use in a dermatological treatment as agent for protecting cells against NLRP3-inflammasome activation caused by ultraviolet radiation and/ or air pollutants.

2. The composition comprising vitamin B12 for its use in a dermatological treatment according to claim 1, wherein the composition is applied on the skin before or after exposure or contact with ultraviolet radiation and/ or air pollutants.

3. The composition comprising vitamin B12 for its use in a dermatological treatment according to anyone or more of claims 1 to 3, wherein the effective amount is selected in the range of 0.0001 wt.-% to 0.1 wt.-%, preferably in the range of 0.001 wt.-% to 0.05 wt.-%, most preferably in the range of 0.001 to 0.025 wt.-%, based on the total weight of the composition.

4. The composition comprising vitamin B12 for its use in a dermatological treatment according to anyone or more of claims 1 to 3, wherein the amount of the composition to be applied to the skin is selected in the range of 0.1 to 3 mg/ cm² skin.

5. The composition comprising vitamin B12 for its use in a dermatological treatment according to anyone or more of claims 1 to 4, wherein the amount of the composition to be applied to the skin is selected in the range of 0.1 to 2 mg/ cm² skin.

6. The composition comprising vitamin B12 for its use in a dermatological treatment according to anyone or more of claims 1 to 5, wherein the amount of the composition to be applied to the skin is selected in the range of 0.5 to 2 mg / cm² skin.

7. The composition comprising vitamin B12 for its use in a dermatological treatment according to anyone or more of claims 1 to 6, wherein the air pollutants are selected from the group of polycyclic aromatic hydrocarbons, volatile organic compounds, oxides, particulate matter, ozone, and cigarette smoke.

8. The composition comprising vitamin B12 for its use in a dermatological treatment according to anyone or more of claims 1 to 6, wherein the ultraviolet radiation is UV-B or UV-A radiation.

9. The composition comprising vitamin B12 for its use in a dermatological treatment according to claim 8, wherein the composition in addition comprises at least one UV filter substance.

10. The composition comprising vitamin B12 for its use in a dermatological treatment according to anyone or more of claims 1 to 9, wherein the composition is a skin care preparation.

11. The composition comprising vitamin B12 for its use in a dermatological treatment according to anyone or more of claims 1 to 10, wherein the cosmetic or dermatological composition is an O/W emulsion comprising an oily phase dispersed in an aqueous phase in the presence of an O/W emulsifier.

12. The composition comprising vitamin B12 for its use in a dermatological treatment according to claim 11, **characterized in that** the O/W emulsifier is potassium cetyl phosphate.

13. A method to prevent or treat skin conditions caused by UV-radiation and/ or air pollution-induced increase in inflammasomes comprising the following steps:
a) providing a topical composition comprising vitamin B12, and
b) applying an amount of said topical composition to human skin or scalp that is sufficient to mitigate the effect of NLRP3 inflammasome activation.

14. A method of treating a disease or disorder in which the NLRP3 signaling contributes to the pathology, and/or symptoms, and/or progression, of said disease/disorder, said method comprising administering an effective amount of vitamin B12 to a subject in need thereof.

15. Vitamin B12 as NLRP3 inflammasome inhibitor.
